# EUROPÄISCHE PATENTSCHRIFT

(11) **EP 3 808 290 B1**
(45) Veröffentlichungstag und Bekanntmachung des Hinweises auf die Patenterteilung: **10.08.2022**
(21) Anmeldenummer: 20202275.2
(22) Anmeldetag: 16.10.2020
(51) Int. Cl.: A61B 17/3201, A61B 17/28, A61B 17/00, A61B 90/00

(54) **MEDIZINISCHES INSTRUMENT UND VERFAHREN ZUM HERSTELLEN EINES MEDIZINISCHEN INSTRUMENTS**
MEDICAL INSTRUMENT AND METHOD OF MANUFACTURING A MEDICAL INSTRUMENT
INSTRUMENT MÉDICAL ET PROCÉDÉ DE FABRICATION D'UN INSTRUMENT MÉDICAL

(30) Priorität: 18.10.2019 DE 102019128181
(43) Veröffentlichungstag der Anmeldung: 21.04.2021
(73) Patentinhaber: Aesculap AG, 78532 Tuttlingen (DE)
(72) Erfinder: Schweitzer, Tom, 78532 Tuttlingen (DE); Ettwein, Pierre, 78234 Engen (DE)
(74) Vertreter: Hoeger, Stellrecht & Partner Patentanwälte mbB

(56) Entgegenhaltungen:
- DE-C1- 3 701 765
- DE-U1-202011 107 977
- US-B1- 6 193 719

## Beschreibung

Die vorliegende Erfindung betrifft ein medizinisches Instrument mit einer ersten Branche und einer zweiten Branche, die in einer Arbeitsstellung des Instruments mit einer Schlussschraube miteinander gekoppelt und um eine gemeinsame Schwenkachse relativ zueinander verschwenkbar sind, wobei eine Längsachse der Schlussschraube die Schwenkachse definiert.

Ferner betrifft die Erfindung ein Verfahren zum Herstellen eines medizinischen Instruments mit einer ersten Branche und einer zweiten Branche, die in einer Arbeitsstellung des Instruments miteinander gekoppelt und um eine gemeinsame Schwenkachse relativ zueinander verschwenkbar sind, wobei die erste Branche und die zweite Branche in einer Montagestellung voneinander getrennt sind und mit einer Schlussschraube miteinander gekoppelt werden, wobei eine Längsachse der Schlussschraube die Schwenkachse definiert. Medizinische Instrumente der eingangs beschriebenen Art sind beispielsweise aus der DE 10 2004 052 515 A1 und der DE 20 2011 107 977 U1 bekannt. Die beiden Branchen der in dieser Offenlegungsschrift beschriebenen Schere sind mit einer Schlussschraube miteinander gekoppelt.

Schraubverbindungen haben im Gegensatz zu Niet- oder Stiftverbindungen einen großen Vorteil, denn sie ermöglichen es, sowohl Fertigungstoleranzen, welche bei der Herstellung der Rohteile auftreten, als auch einen zusätzlichen Verzug, welcher durch einen Härteprozess beim finalen Setzen der Schlussschraube im gehärteten Zustand auftreten kann, auszugleichen. Dadurch kann sowohl ein möglichst kleines Spiel der Branchen und damit von freien Enden derselben, die Maulteile des Instruments bilden, eingestellt werden. Ferner kann auch die erforderliche Präzision des Instruments sichergestellt werden.

Überdies kann auch eine gleichmäßige Betätigungskraft des Instruments, was auch als gleichmäßiger Gang bezeichnet wird, erreicht werden.

Ein Nachteil bei Schraubverbindungen ist insbesondere die übliche Ausgestaltung des Schraubenkopfes, welcher einen Schlitz aufweist, der am fertiggestellten Instrument eine schlecht reinigbare Vertiefung darstellt und unter Umständen neben einer maschinellen Reinigung einen zusätzlichen manuellen Arbeitsschritt erfordert. Eine einfache und sichere Reinigbarkeit von chirurgischen Instrumenten ist jedoch ein immer wichtiger werdendes Thema bei der Aufbereitung von Instrumenten, um diese mehrfach einsetzen zu können.

Es ist daher eine Aufgabe der vorliegenden Erfindung, ein medizinisches Instrument sowie ein Verfahren der eingangs beschriebenen Art so zu verbessern, dass das Instrument insbesondere einfach und sicher reinigbar ist.

Diese Aufgabe wird bei einem medizinischen Instrument der eingangs beschriebenen Art erfindungsgemäß dadurch gelöst, dass an der Schlussschraube ein von dieser in einer Montagestellung abstehendes Einschraubelement angeordnet oder ausgebildet ist, dass das Einschraubelement mindestens eine Einschraubwerkzeugkontaktfläche zum kraft- und/oder formschlüssigen in Eingriff Bringen mit einem Einschraubwerkzeug aufweist und dass das Einschraubelement nach dem Koppeln der ersten Branche und der zweiten Branche miteinander von der Schlussschraube abtrennbar ist.

Die vorgeschlagene Weiterbildung eines medizinischen Instruments der eingangs beschriebenen Art verbessert eine Reinigbarkeit desselben insbesondere dadurch, dass kein Schlitz beispielsweise am Schraubenkopf der Schlussschraube erforderlich ist. Die Einschraubwerkzeugkontaktfläche zum kraft- und/oder formschlüssigen in Eingriff Bringen mit einem Einschraubwerkzeug, den der Schlitz bei herkömmlichen Schlussschrauben bildet, wird nach der Montage der Branchen entfernt. Dies ermöglicht es, beispielsweise den Schraubenkopf völlig glatt und ohne jede Vertiefung auszubilden, ebenso gegenüberliegendes Ende der Schlussschraube. Zudem ist es möglich, das Einschraubelement in einer Form auszubilden, dass insbesondere eine maschinelle Montage des Instruments möglich wird. Dies kann insbesondere durch geeignete Formgebung des Einschraubelements erreicht werden.

Vorteilhaft ist es, wenn die Schlussschraube und das Einschraubelement einstückig, insbesondere monolithisch, ausgebildet sind. Dies ermöglicht es insbesondere, die Schussschraube mit dem Einschraubelement in einem Arbeitsgang herzustellen.

Vorteilhafterweise ist das Einschraubelement an der der Schlussschraube derart angeordnet oder ausgebildet, dass die Längsachse das Einschraubelement durchsetzt. Mit anderen Worten schneidet die Längsachse das Einschraubelement. So kann auf einfache Weise sichergestellt werden, dass ein zum Einschrauben der Schlussschraube erforderliches Anzugsdrehmoment in optimaler Weise parallel zur Längsachse der Schlussschraube eingeleitet werden kann. Insbesondere bildet die Längsachse der Schlussschraube auch eine Längsachse des Einschraubelements.

Günstig ist es, wenn zwischen dem Einschraubelement und der Schlussschraube eine Sollbruchstelle zum Abtrennen des Einschraubelements von der Schlussschraube ausgebildet ist. Eine solche Sollbruchstelle ermöglicht es insbesondere auf einfache Weise, das Einschraubelement nach dem Koppeln der beiden Branchen miteinander von der Schlussschraube zu lösen. Beispielsweise kann so auch die Fläche der Schlussschraube, von der das Einschraubelement ursprünglich absteht, bearbeitet werden, so dass sie glatt oder im Wesentlichen glatt ist und dadurch gut reinigbar, da keine Vertiefungen oder dergleichen in diesem Bereich der Schlussschraube vorgesehen sind.

Auf einfache Weise lässt sich das Einschraubelement von der Schlussschraube lösen, wenn die Sollbruchstelle eine Außenquerschnittsfläche quer, insbesondere senkrecht, zur Längsachse definiert, welche kleiner als eine minimale äußere Querschnittsfläche des Einschraubelements und kleiner als eine minimale äußere Querschnittsfläche der Schlussschraube ist.

Vorteilhaft ist es, wenn die die Schlussschraube einen Schraubenkopf und einen vom Schraubenkopf abstehenden Bolzen umfasst und wenn der Bolzen mindestens einen Außengewindeabschnitt aufweist, welcher sich mindestens über einen Teil einer Länge des Bolzens parallel zur Längsachse erstreckt. Insbesondere kann sich der Außengewindeabschnitt über die gesamte Länge des Bolzens erstrecken. Zwei Branchen lassen sich mit einer derart ausgebildeten Schlussschraube auf einfache Weise miteinander koppeln. Der Bolzen kann mit seinem Außengewindeabschnitt beispielsweise mit einer Branche verschraubt sein. Die andere Branche kann zwischen dem Schraubenkopf und der mit dem Bolzen verschraubten Branche um den Bolzen und damit um die Längsachse der Schlussschraube verschwenkbar gelagert werden.

Günstig ist es, wenn die erste Branche eine erste Durchbrechung aufweist, wenn die zweite Branche eine zweite Durchbrechung aufweist, wenn an der zweiten Durchbrechung ein Innengewinde ausgebildet ist, das zum Außengewindeabschnitt korrespondiert, und wenn in der Arbeitsstellung des medizinischen Instruments der Bolzen die erste Durchbrechung durchsetzt, der Außengewindeabschnitt mit dem Innengewinde verschraubt ist und der Schraubenkopf an einer von der zweiten Branche weg weisenden Seitenfläche der ersten Branche anliegt. Eine solche Ausgestaltung ermöglicht es insbesondere, die erste Branche um den eine Lagerwelle bildenden Bolzen zwischen der zweiten Branche und dem Schraubenkopf verschwenkbar zu lagern.

Vorzugsweise ist der Einschraubkörper derart bemessen, dass er durch die erste und die zweite Durchbrechung hindurchführbar ist. Dies ermöglicht es insbesondere, den Einschraubkörper am Bolzen anzuordnen, so dass nach dem Entfernen des Einschraubelements eine kleinere Fläche bearbeitet werden muss, nämlich eine Endfläche des Bolzens, als eine in entgegengesetzter Richtung weisende Endfläche des Schraubenkopfs. Zudem ist es so möglich, den Schraubenkopf, insbesondere eine Endfläche desselben, bereits bei der Herstellung der Schlussschraube in eine gewünschte und optimal reinigbare Form zu bringen, also beispielsweise glatt, insbesondere ohne Vertiefungen, auszubilden.

Um eine Reinigbarkeit des Instruments weiter zu verbessern, ist es vorteilhaft, wenn an der ersten eine koaxial zur ersten Durchbrechung angeordnete und bezogen auf die Durchbrechung im Querschnitt erweiterte Schraubenkopfausnehmung zum mindestens teilweisen, insbesondere vollständigen, Aufnehmen des Schraubenkopfs ausgebildet ist. Beispielsweise kann die Schraubenkopfausnehmung derart ausgebildet sein, dass sie den Schraubenkopf formschlüssig aufnimmt. Insbesondere kann die Schraubenkopfausnehmung koaxial zur Längsachse der Schlussschraube ausgebildet sein.

Vorteilhaft ist es, wenn der Schraubenkopf einen Schraubenkopfaußendurchmesser quer zur Längsachse definiert, wenn der Bolzen einen Bolzenaußendurchmesser quer zur Längsachse definiert und wenn der Schraubenkopfaußendurchmesser größer ist als der Bolzenaußendurchmesser. Dies ermöglicht es insbesondere wie bereits oben beschrieben, die erste Branche zwischen dem Schraubenkopf und der zweiten Branche, die mit der Schlussschraube verschraubt ist, verschwenkbar in definierter Weise zu lagern.

Günstigerweise definiert der Schraubenkopf eine vom Bolzen weg weisende Schraubenkopfendfläche. Diese kann wie beschrieben insbesondere ohne jegliche Vertiefungen ausgebildet werden, um die Reinigbarkeit des Instruments zu verbessern.

Vorzugsweise ist die Schraubenkopfendfläche glatt oder im Wesentlichen glatt ausgebildet, insbesondere in der Montagestellung oder nach dem Abtrennen des Einschraubelements von der Schlussschraube. Glatt beziehungsweise im Wesentlichen glatt bedeutet hier insbesondere, dass keine Strukturierung, insbesondere weder makroskopisch noch mikroskopisch, der Schraubenkopfendfläche vorgesehen ist, welche schlecht zu reinigen wäre.

Vorteilhaft ist es, wenn die die Schraubenkopfendfläche eben oder vom Schraubenkopf weg weisend konvex oder konkav gekrümmt ausgebildet ist. So kann die Schraubenkopfendfläche insbesondere an eine Außenkontur beziehungsweise Außenfläche der ersten Branche, die von der zweiten Branche weg weist, angepasst werden, beispielsweise um die Schraubenkopfendfläche flächenbündig mit einer Außenfläche der ersten Branche auszubilden.

Günstig ist es, wenn sich die Schraubenkopfendfläche quer, insbesondere senkrecht, zur Längsachse erstreckt. Dies ermöglicht auf einfache Weise eine flächenbündige Anpassung der Schraubenkopfendfläche an eine Außenfläche der ersten Branche.

Ferner ist es vorteilhaft, wenn der der Bolzen eine vom Schraubenkopf weg weisende Bolzenendfläche definiert. Diese kann beliebig gestaltet werden, insbesondere frei von jeder Art von Vertiefungen und dergleichen, um eine Reinigbarkeit des medizinischen Instruments nicht zu beeinträchtigen.

Vorzugsweise ist das Einschraubelement von der Bolzenendfläche abstehend angeordnet oder ausgebildet. Insbesondere dann, wenn die Bolzenendfläche kleiner als die Schraubenkopfendfläche ist, muss so nach dem Abtrennen des Einschraubelements, welches von der Bolzenendfläche abstehend angeordnet oder ausgebildet ist, eine kleinere Fläche bearbeitet werden.

Günstig ist es, wenn die Bolzenendfläche glatt oder im Wesentlichen glatt ausgebildet ist. Insbesondere kann sie bereits in der Montagestellung glatt oder im Wesentlichen glatt ausgebildet sein oder nach dem Abtrennen des Einschraubelements von der Schlussschraube. Die Bolzenendfläche kann insbesondere in der Montagestellung bereits glatt ausgebildet sein, wenn nämlich das Einschraubelement von der Schraubenkopfendfläche abstehend angeordnet oder ausgebildet ist.

Vorteilhafterweise ist die die Bolzenendfläche eben oder vom Bolzen weg weisend konvex oder konkav gekrümmt ausgebildet. Eine derart ausgestaltete Bolzenendfläche ermöglicht es insbesondere, die Bolzenendfläche flächenbündig mit einer von der ersten Branche weg weisenden Seitenfläche der zweiten Branche auszubilden. So kann die Ausbildung von Vertiefungen am Instrument in diesem Bereich verhindert werden, wodurch die Reinigbarkeit des Instruments weiter verbessert wird.

Günstig ist es, wenn sich die Bolzenendfläche quer, insbesondere senkrecht, zur Längsachse erstreckt. Dies ermöglicht auf einfache Weise eine flächenbündige Anpassung der Bolzenendfläche an eine Außenfläche der zweiten Branche.

Gemäß einer weiteren bevorzugten Ausführungsform der Erfindung kann vorgesehen sein, dass in der Arbeitsstellung die Bolzenendfläche flächenbündig mit einer von der ersten Branche weg weisenden Seitenfläche der zweiten Branche angeordnet ist, insbesondere nach dem Abtrennen des Einschraubelements. So kann eine praktisch vollständig glatte durchgehende Seitenfläche der zweiten Branche im Bereich der Bolzenendfläche ausgebildet werden, wodurch eine Reinigbarkeit des Instruments verbessert wird.

Günstig ist es, wenn die mindestens eine Einschraubwerkzeugkontaktfläche von der Schwenkachse weg oder auf diese hin weisend ausgebildet ist. Insbesondere können zwei, drei oder mehr Einschraubwerkzeugkontaktflächen vorgesehen sein. Sind Einschraubwerkzeugkontaktflächen vorgesehen, können diese im Querschnitt quer zur Längsachse der Schlussschraube insbesondere eine viereckige Kontur definieren, beispielsweise in Form einer viereckigen Ausnehmung oder einer viereckigen Außenkontur des Einschraubelements. So kann auf einfache Weise ein Formschluss ausgebildet werden zwischen dem Einschraubwerkzeug und der mindestens einen Einschraubwerkzeugkontaktfläche. Eine einzige Einschraubwerkzeugkontaktfläche kann beispielsweise vorgesehen sein, wenn das Einschraubelement im Querschnitt kreisförmig oder oval ausgebildet ist. So kann bei einem kreisförmigen Querschnitt des Einschraubelements beispielsweise ein kraftschlüssiges in Eingriff Bringen erreicht werden durch eine Klemmverbindung mit dem Einschraubwerkzeug, beispielsweise wenn dieses zwei Backen aufweist. Eine Formschlussverbindung kann insbesondere erreicht werden, wenn das Einschraubwerkzeug korrespondierend zum Einschraubelement ausgebildet ist, insbesondere um einen Formschluss zwischen dem Einschraubelement und dem Einschraubwerkzeug zu realisieren.

Auf einfache Weise ausbilden lässt sich die Schlussschraube, wenn das Einschraubelement symmetrisch bezogen auf die Längsachse oder bezogen auf eine die Längsachse enthaltende Ebene ausgebildet ist.

Vorteilhaft ist es, wenn das Einschraubelement im Bereich der mindestens einen Einschraubwerkzeugkontaktfläche eine äußere Querschnittsfläche quer, insbesondere senkrecht, zur Längsachse definiert und wenn die Querschnittsfläche kreisförmig ist oder eine von einer Kreisform abweichende Form aufweist. Eine kreisförmige Querschnittsfläche ermöglicht insbesondere ein kraftschlüssiges in Eingriff Bringen mit einem entsprechend ausgebildeten Einschraubwerkzeug, beispielsweise einer Zange. Weicht eine Form der Querschnittsfläche von einer Kreisform ab, lässt sich auf einfache Weise ein Formschluss zwischen dem Einschraubelement und dem Einschraubwerkzeug realisieren, um ein Anzugsdrehmoment der Schlussschraube vom Einschraubwerkzeug auf die Schlussschraube zu übertragen.

Auf einfache und kostengünstige Weise lässt sich das medizinische Instrument ausbilden, wenn die von einer Kreisform abweichende Form der äußeren Querschnittsfläche vieleckig, insbesondere dreieckig, viereckig, fünfeckig, sechseckig, sternförmig, oval ist, einen Außenvielrund definiert oder kreisförmig mit mindestens einer Abflachung ist. Beispielsweise können bei einer Kreisform eine, zwei oder mehr Abflachungen vorgesehen sein.

Ferner ist es günstig, wenn das Einschraubelement eine Aufnahme für ein Einschraubwerkzeug definiert und wenn die Aufnahme die mindestens eine Einschraubwerkzeugkontaktfläche definiert. Diese Ausgestaltung ermöglicht es insbesondere, die mindestens eine Einschraubwerkzeugkontaktfläche in Richtung auf die Längsachse hin weisend auszubilden.

Günstig ist es, wenn die Aufnahme ausgebildet ist zum kraft- und/oder formschlüssigen in Eingriff Bringen mit einem Einschraubwerkzeug. Auf diese Weise lassen sich die beiden Branchen mit der Schlussschraube einfach und sicher verbinden.

Eine einfache und sichere formschlüssige Kopplung zwischen dem Einschraubwerkzeug und dem Einschraubelement wird insbesondere dadurch ermöglicht, dass die Aufnahme in Form eines Schlitzes oder einer Ausnehmung ausgebildet ist. Insbesondere kann die Ausnehmung in Form eines Sacklochs ausgebildet sein.

Gemäß einer weiteren bevorzugten Ausführungsform der Erfindung kann vorgesehen sein, dass die Aufnahme eine innere Querschnittsfläche im Bereich der mindestens einen Einschraubwerkzeugkontaktfläche quer, insbesondere senkrecht, zur Längsachse definiert und dass die innere Querschnittsfläche kreisförmig ist oder eine von einer Kreisform abweichende Form aufweist. Eine solche Aufnahme ermöglicht es insbesondere, dass ein korrespondierend ausgebildetes Ende eines Einschraubwerkzeugs formschlüssig in die Aufnahme eingreifen kann, um ein Anzugsdrehmoment auf die Schlussschraube zu übertragen.

Vorteilhaft ist es, wenn die von einer Kreisform abweichende Form der inneren Querschnittsfläche vieleckig, insbesondere dreieckig, viereckig, fünfeckig, sechseckig, sternförmig, oval ist, einen Innenvielrund definiert oder kreisförmig mit mindestens einer Abflachung. Derartige Querschnittsformen der Aufnahme lassen sich auf einfache Weise ausbilden. Insbesondere ermöglichen sie es, herkömmliche Einschraubwerkzeuge zu nutzen, so dass keine Spezialwerkzeuge hierfür erforderlich sind.

Ferner ist es günstig, wenn die erste Branche mindestens ein erstes Werkzeugelement umfasst, wenn die die zweite Branche mindestens ein zweites Werkzeugelement umfasst und wenn das mindestens eine erste Werkzeugelement und das mindestens eine zweite Werkzeugelement miteinander zusammenwirkend angeordnet sind zur Ausbildung eines medizinischen Werkzeugs. Die beiden Werkzeugelemente werden üblicherweise auch als Maulteile des Instruments bezeichnet. Beispielsweise können zwei Schneidelemente zusammenwirken, um ein Instrument in Form einer Schere auszubilden.

Um insbesondere Scheren, Nadelhalter oder Klemmen ausbilden zu können, ist es vorteilhaft, wenn das mindestens eine erste Werkzeugelement in Form einer Schneide oder eines Klemmbackens ausgebildet ist und/oder wenn das mindestens eine zweite Werkzeugelement in Form einer Schneide oder eines Klemmbackens ausgebildet ist.

Vorzugsweise ist das medizinische Instrument in Form einer Fasszange, einer Schere, eines Nadelhalters oder einer Klemme ausgebildet. Die Reinigbarkeit derartiger Instrumente lässt sich in der beschriebenen Weise einfach und sicher verbessern.

Die eingangs gestellte Aufgabe wird ferner bei einem Verfahren der eingangs beschriebenen Art erfindungsgemäß dadurch gelöst, dass zum Koppeln der ersten Branche und der zweiten Branche eine Schlussschraube eingesetzt wird, an der ein von dieser in der Montagestellung abstehendes Einschraubelement angeordnet oder ausgebildet ist, dass das Einschraubelement mindestens eine Einschraubwerkzeugkontaktfläche zum kraft- und/oder formschlüssigen in Eingriff Bringen mit einem Einschraubwerkzeug aufweist, dass zum Koppeln der ersten Branche und der zweiten Branche mit der Schlussschraube das Einschraubwerkzeug mit der mindestens einen Einschraubwerkzeugkontaktfläche kraft- und/oder formschlüssig in Eingriff gebracht wird und dass das Einschraubelement nach dem Koppeln der ersten Branche und der zweiten Branche von der Schlussschraube abgetrennt wird.

Ein Verfahren zum Herstellen eines medizinischen Instruments in der beschriebenen Weise weiterzubilden ermöglicht es insbesondere, Endflächen der Schlussschraube, beispielsweise eine oben beschriebene Bolzenendfläche oder eine oben beschriebene Schraubenkopfendfläche, vollständig glatt auszubilden, beispielsweise bündig mit Seitenflächen der Branchen in der Arbeitsstellung anzuordnen, wodurch keine Vertiefungen oder Ausnehmungen gebildet werden, die schlecht reinigbar sind. Zudem kann ein solches Verfahren auf einfache Weise auch maschinell realisiert werden, wenn das Einschraubelement eine Form aufweist, die ein maschinelles in Eingriff Bringen gestattet. Ferner wird die Verwendung eines solchen Verfahrens zum Herstellen eines der oben beschriebenen medizinischen Instrumente vorgeschlagen.

Die nachfolgende Beschreibung bevorzugter Ausführungsformen der Erfindung dient im Zusammenhang mit den Zeichnungen der näheren Erläuterung. Es zeigen:
- Figur 1:: eine perspektivische Gesamtansicht eines medizinischen Instruments aus dem Stand der Technik;
- Figur 2:: eine vergrößerte Teilansicht des Bereichs A aus Figur 1 bei gegeneinander verschwenkten Branchen;
- Figur 3:: eine schematische, teilweise durchbrochene Darstellung eines Ausschnitts ähnlich des Bereichs A aus Figur 1 eines Ausführungsbeispiels eines erfindungsgemäßen medizinischen Instruments;
- Figur 4:: eine Ansicht der Anordnung aus Figur 3 in Richtung des Pfeils B;
- Figur 5:: eine schematische Darstellung eines vorderen Teils eines weiteren Ausführungsbeispiels eines medizinischen Instruments;
- Figur 6:: eine Ansicht ähnlich Figur 4 eines weiteren Ausführungsbeispiels eines medizinischen Instruments;
- Figur 7:: eine schematische Schnittansicht eines Schlussbereichs eines Ausführungsbeispiels eines medizinischen Instruments;
- Figur 8:: eine Schnittansicht längs Linie 7-7 in Figur 7;
- Figur 9:: eine Schnittansicht analog Linie 7-7 in Figur 7 eines weiteren Ausführungsbeispiels eines Einschraubelements;
- Figur 10:: eine Schnittansicht analog Linie 7-7 in Figur 7 eines weiteren Ausführungsbeispiels eines Einschraubelements;
- Figur 11:: eine Schnittansicht analog Linie 7-7 in Figur 7 eines weiteren Ausführungsbeispiels eines Einschraubelements;
- Figur 12:: eine Schnittansicht analog Linie 7-7 in Figur 7 eines weiteren Ausführungsbeispiels eines Einschraubelements;
- Figur 13:: eine Schnittansicht analog Linie 7-7 in Figur 7 eines weiteren Ausführungsbeispiels eines Einschraubelements;
- Figur 14:: eine Schnittansicht analog Linie 7-7 in Figur 7 eines weiteren Ausführungsbeispiels eines Einschraubelements;
- Figur 15:: eine Schnittansicht analog Linie 7-7 in Figur 7 eines weiteren Ausführungsbeispiels eines Einschraubelements;
- Figur 16:: eine Schnittansicht analog Linie 7-7 in Figur 7 eines weiteren Ausführungsbeispiels eines Einschraubelements;
- Figur 17:: eine schematische Schnittansicht des Schlussbereichs eines weiteren Ausführungsbeispiels eines medizinischen Instruments in der Montagestellung; und
- Figur 18:: eine schematische Schnittansicht des Schlussbereichs eines medizinischen Instruments in der Arbeitsstellung.

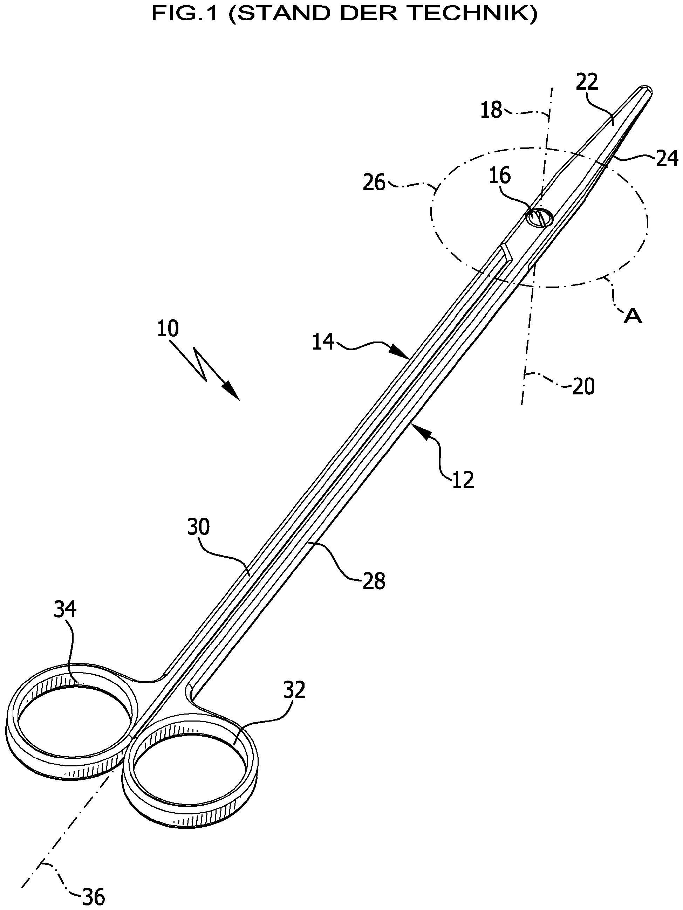

Ein aus dem Stand der Technik bekanntes Ausführungsbeispiel eines medizinischen Instruments 10 mit einer ersten Branche 12 und einer zweiten Branche 14 ist schematisch in Figur 1 in Form einer Schere dargestellt.

In der in Figur 1 dargestellten Arbeitsstellung des Instruments 10 sind die Branchen 12 und 14 mit einer Schlussschraube 16 miteinander gekoppelt und um eine gemeinsame Schwenkachse 18 relativ zueinander verschwenkbar. Eine Längsachse 20 der Schlussschraube 16 definiert die Schwenkachse 18.

Distale Enden der Branchen 12 und 14 sind in Form von Werkzeugelementen 22 und 24 ausgebildet.

Proximalseitig des in Figur 1 durch den Bereich A definierten Schlussbereichs 26 erstrecken sich in einer geschlossenen Stellung des Instruments 10 zwei parallel zueinander verlaufende langgestreckte Halme 28 und 30, an deren freien Enden sich Augen 32 und 34 anschließen. Die Augen 32 und 34 sind ringförmig ausgebildet. Die aneinander anliegenden Halme 28 und 30 definieren eine Instrumentenlängsachse 36.

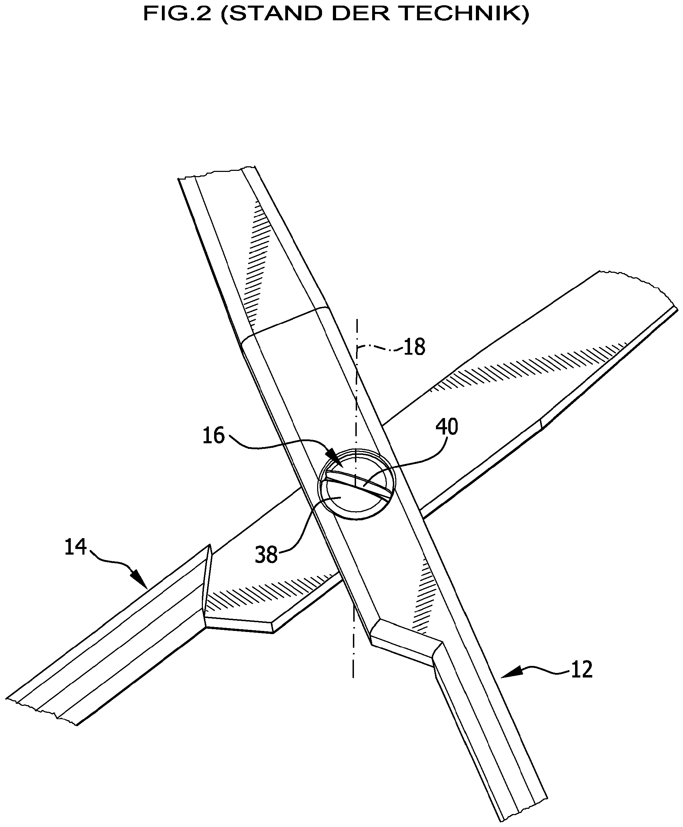

Figur 2 zeigt vergrößert den Schlussbereich 26 mit der Schlussschraube 16, die in herkömmlicher Weise einen vom Schraubenkopf 38 weg weisend geöffneten Schraubenschlitz 40 aufweist. Dieser ist wie oben beschrieben häufig maschinell nicht optimal reinigbar, so dass eine manuelle Nachreinigung insbesondere des Schraubenkopfs 38 mit dem Schraubenschlitz 40 erforderlich ist.

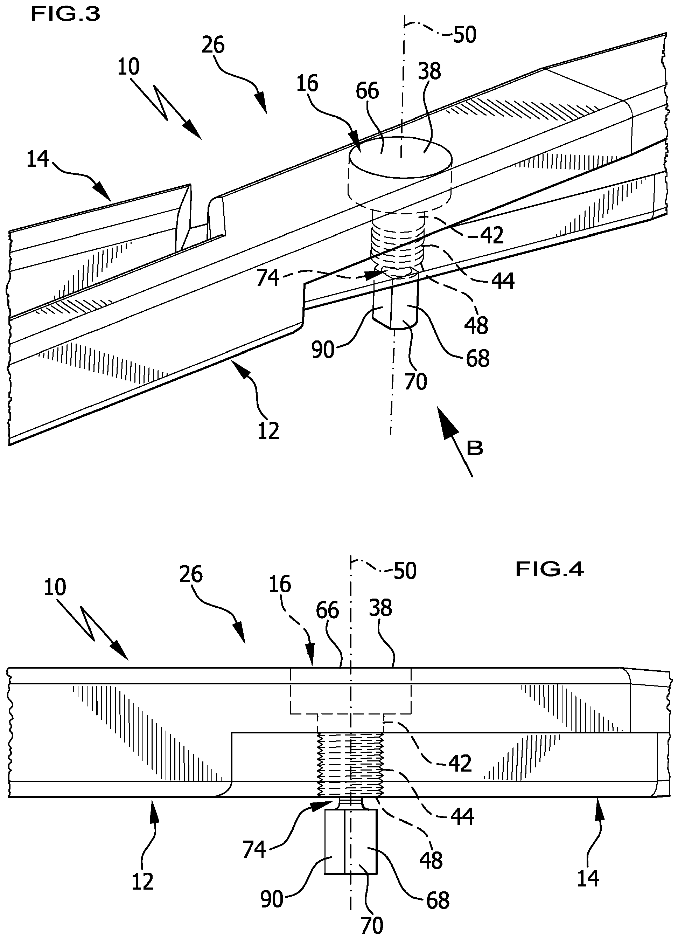

Um die Reinigbarkeit des medizinischen Instruments 10 zu verbessern, wird die Schlussschraube 16, wie bei der schematisch in Figur 3 dargestellten Ansicht des Schlussbereichs 26 eines Ausführungsbeispiels eines Instruments 10 gemäß der Erfindung, mit einem Schraubenkopf 38 ohne Schraubenschlitz ausgebildet.

Die Schlussschraube 16 umfasst einen Schraubenkopf 38 und einen von diesem abstehenden Bolzen 42.

Der Bolzen 42 weist einen Außengewindeabschnitt 44 auf, welcher sich ausgehend von einer vom Schraubenkopf 38 weg weisenden Bolzenendfläche 48 in Richtung auf den Schraubenkopf 38 hin über einen Teil der Länge des Bolzens 42 erstreckt.

Die Schlussschraube 16 definiert eine Längsachse 50.

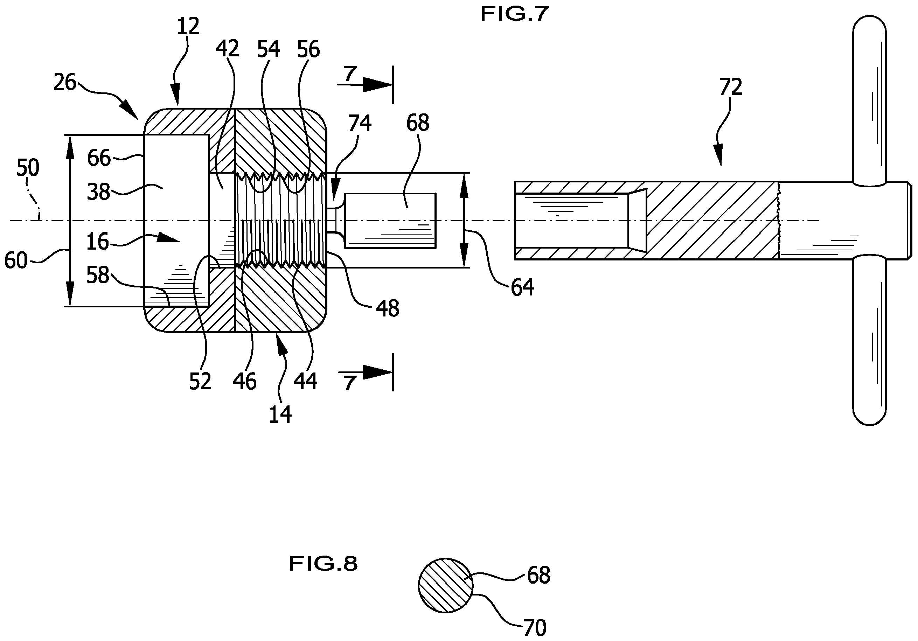

Figur 7 zeigt eine schematische Teilschnittansicht des Schlussbereichs 26 der Figur 3.

Die erste Branche 12 weist eine erste Durchbrechung 52 in Form einer Bohrung auf. Die zweite Branche 14 weist eine zweite Durchbrechung 44 auf, an welcher ein Innengewinde 56 ausgebildet ist, das zum Außengewindeabschnitt 44 korrespondiert.

Die erste Durchbrechung 52 ist so bemessen, dass der Bolzen 42 mit der Bolzenendfläche 48 voran durch die erste Durchbrechung 52 hindurchgeführt und mit dem Außengewindeabschnitt 44 in die zweite Branche 14 eingeschraubt werden kann.

An der ersten Branche 12 ist eine koaxial zur ersten Durchbrechung 52 angeordnete und bezogen auf diese im Querschnitt erweiterte Schraubenkopfausnehmung 58 zum Aufnehmen des Schraubenkopfs 38 ausgebildet.

Der Schraubenkopf 38 definiert einen Schraubenkopfaußendurchmesser 60 quer zur Längsachse 50. Der Bolzen 42 definiert einen Bolzenaußendurchmesser 64 quer zur Längsachse 50 derart, dass der Schraubenkopfaußendurchmesser 60 größer ist als der Bolzenaußendurchmesser 64.

Der Schraubenkopf 38 definiert eine vom Bolzen 42 weg weisende Schraubenkopfendfläche 66. Die Schraubenkopfendfläche 66 und die Bolzenendfläche 48 weisen in entgegengesetzte Richtungen.

In einer in den Figuren 3 und 7 schematisch dargestellten Montagestellung ist an der Schlussschraube 16 ein von dieser abstehendes Einschraubelement 68 angeordnet beziehungsweise ausgebildet, welches mehrere, nämlich vier Einschraubwerkzeugkontaktflächen 70 aufweist, die von der Längsachse 50 weg weisen.

Die Einschraubwerkzeugkontaktflächen 70 sind ausgebildet zum kraft- und/oder formschlüssigen in Eingriff Bringen mit einem in Figur 7 schematisch dargestellten Einschraubwerkzeug 72.

Die Schlussschraube 16 und das Einschraubelement 68 sind einstückig, nämlich monolithisch, ausgebildet.

Bei alternativen Ausführungsbeispielen kann das Einschraubelement 68 an die Schlussschraube 16 nachträglich angeformt sein, beispielsweise durch selektives Lasersintern.

Das Einschraubelement 68 ist an der Schlussschraube 16 derart angeordnet, dass die Längsachse 50 das Einschraubelement 68 durchsetzt.

Das Einschraubelement 68 ist nach dem Koppeln der Branchen 12 und 14 von der Schlussschraube 16 abtrennbar. Um dies zu erleichtern, ist zwischen dem Einschraubelement 68 und der Schlussschraube 16 eine Sollbruchstelle 74 ausgebildet.

Die Sollbruchstelle 74 bildet eine Art Einschnürung zwischen dem Einschraubelement 68 und der Schlussschraube 16 derart, dass die Sollbruchstelle eine Außenquerschnittsfläche 76 quer, nämlich senkrecht, zur Längsachse 50 definiert, welche kleiner ist als eine minimale äußere Querschnittsfläche 78 des Einschraubelements 68 und kleiner als eine minimale äußere Querschnittsfläche 80 der Schlussschraube 16 im Bereich des Schraubenkopfs 38 beziehungsweise kleiner als eine minimale äußere Querschnittsfläche der Schlussschraube 16 im Bereich des Bolzens 42.

Bei dem in den Figuren 3 und 7 dargestellten Ausführungsbeispiel ist das Einschraubelement 68 von der Bolzenendfläche 48 abstehend angeordnet beziehungsweise ausgebildet.

Nach dem Abtrennen des Einschraubelements 68 von der Schlussschraube 16 ist die Bolzenendfläche 48 glatt oder im Wesentlichen glatt ausgebildet. Hierzu kann sie nach dem Abtrennen des Einschraubelements 68 von der Bolzenendfläche 48 entsprechend bearbeitet werden, beispielsweise durch Glattschleifen.

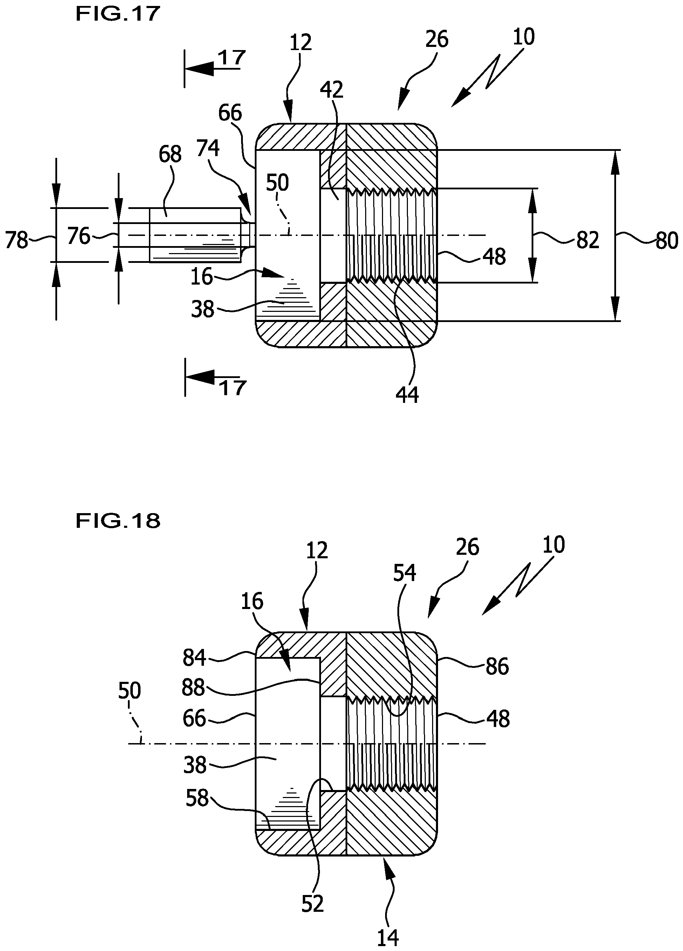

Figur 18 zeigt schematisch den Schlussbereich 26 des Instruments 10 nach dem Abtrennen des Einschraubelements 68. Sowohl die Bolzenendfläche 48 als auch die Schraubenkopfendfläche 66 sind glatt und eben ausgebildet. Sowohl die Bolzenendfläche 48 als auch die Schraubenkopfendfläche 66 erstrecken sich quer zur Längsachse 50.

In Figur 18 ist insbesondere gut zu erkennen, dass in der Arbeitsstellung die Schraubenkopfendfläche 66 und die Bolzenendfläche 48 flächenbündig beziehungsweise im Wesentlichen flächenbündig mit einer Seitenfläche der ersten Branche 12, die von der zweiten Branche 14 weg weist, beziehungsweise mit einer Seitenfläche 86 der zweiten Branche 14, die von der ersten Branche 12 weg weist, angeordnet sind.

In der Arbeitsstellung liegt der Schraubenkopf 38 an einer von der zweiten Branche 14 weg weisenden Seitenfläche 88 der ersten Branche 12 an. Diese Seitenfläche 88 wird durch eine die erste Durchbrechung 52 umgebende und von der zweiten Branche 14 weg weisende Ringfläche gebildet. Diese Ringfläche begrenzt die Schraubenkopfausnehmung 58.

Um die Montage des Instruments 10 in der beschriebenen Weise zu ermöglichen, ist der Einschraubkörper 68 derart bemessen, dass er durch die erste und die zweite Durchbrechung 52, 54 hindurchführbar ist.

Alternativ ist das Einschraubelement 68 bei einem anderen Ausführungsbeispiel eines Instruments 10, dessen Schlussbereich 26 schematisch in Figur 17 dargestellt ist, von der Schraubenkopfendfläche 66 abstehend angeordnet beziehungsweise ausgebildet.

Nach dem Abtrennen des Einschraubelements 68 nach dem Koppeln der beiden Branchen 12 und 14 wird die Schraubenkopfendfläche 66 wiederum so bearbeitet, dass sie glatt oder im Wesentlichen glatt ist. Die Schraubenkopfendfläche 66 ist bei diesem Ausführungsbeispiel eben ausgebildet. Bei anderen Ausführungsbeispielen kann die Schraubenkopfendfläche 66 vom Schraubenkopf weg weisend konvex oder konkav gekrümmt ausgebildet sein. In analoger Weise kann bei Ausführungsbeispielen auch die Bolzenendfläche 48 vom Bolzen 42 weg weisend konvex oder konkav gekrümmt ausgebildet sein.

Bei Ausführungsbeispielen der Schlussschraube 16, bei denen das Einschraubelement 68 von der Schraubenkopfendfläche 66 abstehend angeordnet oder ausgebildet ist, kann dieses eine äußere Querschnittsfläche 78 aufweisen, die größer ist als ein freier Querschnitt, der durch die Durchbrechungen 52 und 54 definiert ist, da bei dieser Variante das Einschraubelement 68 nicht durch die Durchbrechungen 52 und 54 beim miteinander Koppeln der Branchen 12 hindurchgeführt werden muss.

Das Einschraubelement 68 kann auf verschiedene Arten und Weisen ausgebildet sein.

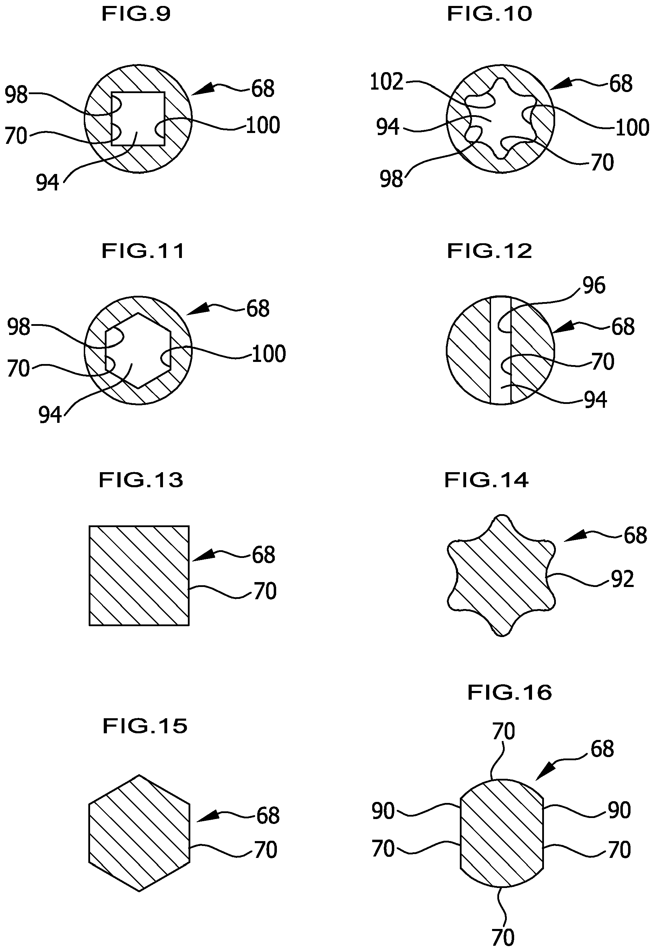

In den Figuren 3 und 4 ist ein Ausführungsbeispiel einer Schlussschraube 16 mit einem Einschraubelement 68 dargestellt, welches einen kreisförmigen Querschnitt mit zwei parallel zueinander verlaufenden Abflachungen 90 aufweist. Ein solcher Querschnitt ist schematisch in Figur 16 dargestellt.

Die vier Einschraubwerkzeugkontaktflächen 70 weisen bei diesem Ausführungsbeispiel von der Schwenkachse 18 weg und umfassen zwei parallele Flächenabschnitte und zwei von der Schwenkachse 18 weg weisend konvex gekrümmte Einschraubwerkzeugkontaktflächen 70 auf.

Weitere Ausführungsbeispiele von Einschraubelementen 68 mit von der Schwenkachse 18 weg weisenden Einschraubwerkzeugkontaktflächen 70 sind in den Figuren 8 sowie 13 bis 15 dargestellt.

Ein kreisförmiger Querschnitt ist in Figur 8 dargestellt, ein viereckiger Querschnitt in Figur 13, eine Querschnittsfläche eines Außenvielrunds in Figur 14 sowie eine hexagonale Querschnittsfläche in Figur 15.

Bei alternativen Ausführungsbeispielen definiert das Einschraubelement 68 eine vom Bolzen 42 weg weisend geöffnete Aufnahme 94 für ein korrespondierendes Einschraubwerkzeug 72, wobei die Aufnahme 94 eine oder mehrere Einschraubwerkzeugkontaktflächen 70 definiert.

Die Aufnahme 94 ist zum kraft- und/oder formschlüssigen in Eingriff Bringen mit dem Einschraubwerkzeug 72 ausgebildet. Dieses weist in nicht dargestellter Form ein Ende auf, welches korrespondierend zur Aufnahme 94 geformt ist.

Die Aufnahme 94 ist bei einem Ausführungsbeispiel in Form eines Schlitzes, wie schematisch in Figur 12 dargestellt, oder in Form einer Ausnehmung 98 ausgebildet, wie schematisch bei den Ausführungsbeispielen der Figuren 9, 10 und 11.

Die Ausnehmung 98 ist bei Ausführungsbeispielen in Form eines Sacklochs 100 ausgebildet.

Die Aufnahme 94 definiert eine innere Querschnittsfläche im Bereich der ein oder mehreren Einschraubwerkzeugkontaktflächen 70 quer, nämlich senkrecht, zur Längsachse 50. Die innere Querschnittsfläche ist, wie in den Figuren nicht dargestellt kreisförmig, oder weist eine von einer Kreisform abweichende Form auf, beispielsweise viereckig wie schematisch in Figur 9 dargestellt, sechseckig, wie schematisch in Figur 11 dargestellt, oder definiert einen Innenvielrund 102.

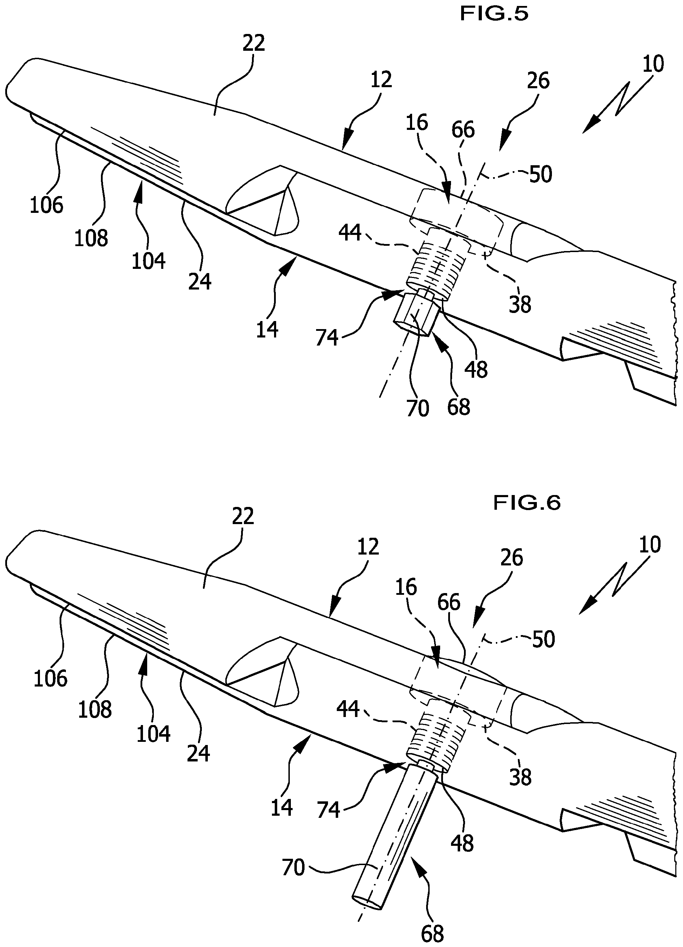

Das schematisch in Figur 5 teilweise dargestellte Ausführungsbeispiel eines Instruments 10 umfasst ein Einschraubelement 68 mit einem sechseckigen Querschnitt.

Das in Figur 6 schematisch teilweise dargestellte Ausführungsbeispiel des Instruments 10 umfasst ein Einschraubelement 68 mit einem kreisförmigen Querschnitt.

Bei dem Instrument 10 können die Werkzeugelemente 22 und 24 in verschiedenen Formen ausgebildet sein. Sie sind in jedem Fall zusammenwirkend angeordnet zur Ausbildung eines medizinischen Werkzeugs 104.

Bei dem in Figur 5 dargestellten Ausführungsbeispiel sind beispielhaft Werkzeugelemente 22 und 24 in Form von Schneiden 106 und 108 ausgebildet, die somit ein Werkzeug 104 zum Schneiden ausbilden.

Die Werkzeugelemente 22 und 24 können bei anderen Ausführungsbeispielen auch in Form von Klemmbacken ausgebildet sein. Ein Instrument 10 mit derartigen Werkzeugelementen 22 und 24 kann insbesondere in Form einer Fasszange, eines Nadelhalters oder einer Klemme ausgebildet sein.

Ein Instrument mit zwei Schneiden 106 und 108 kann insbesondere in Form einer Schere ausgebildet sein.

Die beschriebenen Ausführungsbeispiele von Instrumenten 10 können insbesondere derart ausgebildet werden, dass die Branchen 12 und 14 in einer Montagestellung voneinander getrennt sind. Sie werden mit der Schlussschraube 16 miteinander gekoppelt. Die Schlussschraube 16 ist wie beschrieben derart ausgebildet, dass an dieser ein in der Montagestellung abstehendes Einschraubelement 68 angeordnet oder ausgebildet ist.

Zum Einschrauben der Schlussschraube 16 mit dem Außengewindeabschnitt 44 in das Innengewinde 46 der zweiten Branche 14 wird das Einschraubelement 68 mit dem Einschraubwerkzeug 72 kraft- und/oder formschlüssig in Eingriff gebracht, bis die Branchen 12 und 14 in gewünschter und definierter Weise miteinander gekoppelt sind.

Dann wird das Einschraubelement 68 von der Schlussschraube 16 abgetrennt und die jeweilige Fläche der Schlussschraube 16, also entweder die Bolzenendfläche 48 oder die Schraubenkopfendfläche 66, wird bearbeitet, bis diese glatt ist und keine Aufnahmen mehr aufweist.

Die beschriebenen Ausführungsbeispiele von Instrumenten 10 sind im Vergleich zu Instrumenten 10 aus dem Stand der Technik, welche Schlussschrauben 16 mit einem Schraubenschlitz 40 aufweisen, deutlich leichter reinigbar.

Die beschriebenen Schlussschrauben 16 ermöglichen somit nicht nur eine gute Reinigbarkeit, sondern auch das Ausbilden von Instrumenten 10 mit definiert geringerem Gelenkspiel, gleichmäßiger Betätigungskraft beziehungsweise gleichmäßigem Gang und ermöglichen zudem eine prozesssichere Montage.

### Bezugszeichenliste

- 10: Instrument
- 12: erste Branche
- 14: zweite Branche
- 16: Schlussschraube
- 18: Schwenkachse
- 20: Längsachse
- 22: Werkzeugelement
- 24: Werkzeugelement
- 26: Schlussbereich
- 28: Halm
- 30: Halm
- 32: Auge
- 34: Auge
- 36: Instrumentenlängsachse
- 38: Schraubenkopf
- 40: Schraubenschlitz
- 42: Bolzen
- 44: Außengewindeabschnitt
- 46: Innengewinde
- 48: Bolzenendfläche
- 50: Längsachse
- 52: erste Durchbrechung
- 54: zweite Durchbrechung
- 56: Innengewinde
- 58: Schraubenkopfausnehmung
- 60: Schraubenkopfaußendurchmesser
- 64: Bolzenaußendurchmesser
- 66: Schraubenkopfendfläche
- 68: Einschraubelement
- 70: Einschraubwerkzeugkontaktfläche
- 72: Einschraubwerkzeug
- 74: Sollbruchstelle
- 76: Außenquerschnittsfläche
- 78: Querschnittsfläche
- 80: Querschnittsfläche
- 82: Querschnittsfläche
- 84: Seitenfläche
- 86: Seitenfläche
- 88: Seitenfläche
- 90: Abflachung
- 92: Außenvielrund
- 94: Aufnahme
- 96: Schlitz
- 98: Ausnehmung
- 100: Sackloch
- 102: Innenvielrund
- 104: Werkzeug
- 106: Schneide
- 108: Schneide

## Patentansprüche

1. Medizinisches Instrument (10) mit einer ersten Branche (12) und einer zweiten Branche (14), die in einer Arbeitsstellung des Instruments (10) mit einer Schlussschraube (16) miteinander gekoppelt und um eine gemeinsame Schwenkachse (18) relativ zueinander verschwenkbar sind, wobei eine Längsachse (20) der Schlussschraube (16) die Schwenkachse (18) definiert, **dadurch gekennzeichnet, dass** an der Schlussschraube (16) ein von dieser in einer Montagestellung abstehendes Einschraubelement (68) angeordnet oder ausgebildet ist, dass das Einschraubelement (68) mindestens eine Einschraubwerkzeugkontaktfläche (70) zum kraft- und/oder formschlüssigen in Eingriff Bringen mit einem Einschraubwerkzeug (72) aufweist und dass das Einschraubelement (68) nach dem Koppeln der ersten Branche (12) und der zweiten Branche (14) miteinander von der Schlussschraube (16) abtrennbar ist.

2. Medizinisches Instrument nach Anspruch 1, **dadurch gekennzeichnet, dass** die Schlussschraube (16) und das Einschraubelement (68) einstückig, insbesondere monolithisch, ausgebildet sind.

3. Medizinisches Instrument nach einem der voranstehenden Ansprüche, **dadurch gekennzeichnet, dass** das Einschraubelement (68) an der Schlussschraube (16) derart angeordnet oder ausgebildet ist, dass die Längsachse (50) das Einschraubelement (68) durchsetzt.

4. Medizinisches Instrument nach einem der voranstehenden Ansprüche, **dadurch gekennzeichnet, dass** zwischen dem Einschraubelement (68) und der Schlussschraube (16) eine Sollbruchstelle (74) zum Abtrennen des Einschraubelements (68) von der Schlussschraube (16) ausgebildet ist,
wobei insbesondere die Sollbruchstelle (74) eine Außenquerschnittsfläche (76) quer, insbesondere senkrecht, zur Längsachse (50) definiert, welche kleiner als ein minimale äußere Querschnittsfläche (78) des Einschraubelements (68) und kleiner als eine minimale äußere Querschnittsfläche (80; 82) der Schlussschraube (16) ist.

5. Medizinisches Instrument nach einem der voranstehenden Ansprüche, **dadurch gekennzeichnet, dass** die Schlussschraube (16) einen Schraubenkopf (38) und einen vom Schraubenkopf (38) abstehenden Bolzen (42) umfasst und dass der Bolzen (42) mindestens einen Außengewindeabschnitt (44) aufweist, welcher sich mindestens über einen Teil einer Länge des Bolzens (42) parallel zur Längsachse (50), insbesondere über die gesamte Länge des Bolzens (42), erstreckt.

6. Medizinisches Instrument nach Anspruch 5, **dadurch gekennzeichnet, dass** die erste Branche (12) eine erste Durchbrechung (52) aufweist, dass die zweite Branche (14) eine zweite Durchbrechung (54) aufweist, dass an der zweiten Durchbrechung (54) ein Innengewinde (56) ausgebildet ist, das zum Außengewindeabschnitt (44) korrespondiert, und dass in der Arbeitsstellung des medizinischen Instruments (10) der Bolzen (42) die erste Durchbrechung (52) durchsetzt, der Außengewindeabschnitt (44) mit dem Innengewinde (56) verschraubt ist und der Schraubenkopf (38) an einer von der zweiten Branche (14) weg weisenden Seitenfläche (88) der ersten Branche (12) anliegt.

7. Medizinisches Instrument nach Anspruch 6, **dadurch gekennzeichnet, dass**
a) der Einschraubkörper (68) derart bemessen ist, dass er durch die erste und die zweite Durchbrechung (52, 54) hindurchführbar ist, und/oder
b) an der ersten Branche (12) eine koaxial zur ersten Durchbrechung (52) angeordnete und bezogen auf die erste Durchbrechung (52) im Querschnitt erweiterte Schraubenkopfausnehmung (58) zum mindestens teilweisen, insbesondere vollständigen Aufnehmen des Schraubenkopfs (38) ausgebildet ist.

8. Medizinisches Instrument nach einem der Ansprüche 5 bis 7, **dadurch gekennzeichnet, dass** der Schraubenkopf (38) einen Schraubenkopfaußendurchmesser (60) quer zur Längsachse (50) definiert, dass der Bolzen (42) einen Bolzenaußendurchmesser (64) quer zur Längsachse (50) definiert und dass der Schraubenkopfaußendurchmesser (60) größer ist als der Bolzenaußendurchmesser (64).

9. Medizinisches Instrument nach einem der Ansprüche 5 bis 8, **dadurch gekennzeichnet, dass** der Schraubenkopf (38) eine vom Bolzen (42) weg weisende Schraubenkopfendfläche (66) definiert,
wobei insbesondere
a) das Einschraubelement (68) von der Schraubenkopfendfläche (66) abstehend angeordnet oder ausgebildet ist
und/oder
b) die Schraubenkopfendfläche (66) glatt oder im Wesentlichen glatt ausgebildet ist, insbesondere in der Montagestellung oder nach dem Abtrennen des Einschraubelements (68) von der Schlussschraube (66)
und/oder
c) die Schraubenkopfendfläche (66) eben oder vom Schraubenkopf (38) weg weisend konvex oder konkav gekrümmt ausgebildet ist und/oder
d) sich die Schraubenkopfendfläche (66) quer, insbesondere senkrecht, zur Längsachse (50) erstreckt,
und/oder
e) in der Arbeitsstellung die Schraubenkopfendfläche (66) flächenbündig mit einer von der zweiten Branche (14) weg weisenden Seitenfläche der ersten Branche (12) angeordnet ist, insbesondere nach dem Abtrennen des Einschraubelements (68).

10. Medizinisches Instrument nach einem der Ansprüche 5 bis 9, **dadurch gekennzeichnet, dass** der Bolzen (42) eine vom Schraubenkopf (38) weg weisende Bolzenendfläche (48) definiert,
wobei insbesondere
a) das Einschraubelement (68) von der Bolzenendfläche (48) abstehend angeordnet oder ausgebildet ist
und/oder
b) die Bolzenendfläche (48) glatt oder im Wesentlichen glatt ausgebildet ist, insbesondere in der Montagestellung oder nach dem Abtrennen des Einschraubelements (68) von der Schlussschraube (16),
und/oder
c) die Bolzenendfläche (48) eben oder vom Bolzen (42) weg weisend konvex oder konkav gekrümmt ausgebildet ist
und/oder
d) sich die Bolzenendfläche (48) quer, insbesondere senkrecht, zur Längsachse (50) erstreckt
und/oder
e) in der Arbeitsstellung die Bolzenendfläche (48) flächenbündig mit einer von der ersten Branche (12) weg weisenden Seitenfläche (86) der zweiten Branche (14) angeordnet ist, insbesondere nach dem Abtrennen des Einschraubelements (68).

11. Medizinisches Instrument nach einem der voranstehenden Ansprüche, **dadurch gekennzeichnet, dass**
a) die mindestens eine Einschraubwerkzeugkontaktfläche (70) von der Schwenkachse (18) weg oder auf diese hin weisend ausgebildet ist und/oder
b) das Einschraubelement (68) symmetrisch bezogen auf die Längsachse (50) oder bezogen auf eine die Längsachse (50) enthaltende Ebene ausgebildet ist
und/oder
c) das Einschraubelement (68) im Bereich der mindestens einen Einschraubwerkzeugkontaktfläche (70) eine äußere Querschnittsfläche quer, insbesondere senkrecht, zur Längsachse (50) definiert und dass die Querschnittsfläche kreisförmig ist oder eine von einer Kreisform abweichende Form aufweist,
wobei insbesondere die von einer Kreisform abweichende Form der äußeren Querschnittsfläche vieleckig, insbesondere dreieckig, viereckig, fünfeckig, sechseckig, sternförmig, oval ist, einen Außenvielrund (92) definiert oder kreisförmig mit mindestens einer Abflachung (90).

12. Medizinisches Instrument nach einem der voranstehenden Ansprüche, **dadurch gekennzeichnet, dass** das Einschraubelement (68) eine Aufnahme (94) für ein Einschraubwerkzeug (72) definiert und dass die Aufnahme (94) die mindestens eine Einschraubwerkzeugkontaktfläche (70) definiert,
wobei insbesondere die Aufnahme (94)
a) ausgebildet ist zum kraft- und/oder formschlüssigen in Eingriff Bringen mit einem Einschraubwerkzeug (72)
und/oder
b) in Form eines Schlitzes (96) oder einer Ausnehmung (98), insbesondere in Form eines Sacklochs (100), ausgebildet ist
und/oder
c) eine innere Querschnittsfläche im Bereich der mindestens einen Einschraubwerkzeugkontaktfläche (70) quer, insbesondere senkrecht, zur Längsachse (50) definiert und dass die innere Querschnittsfläche kreisförmig ist oder eine von einer Kreisform abweichende Form aufweist,
wobei insbesondere die von einer Kreisform abweichende Form der inneren Querschnittsfläche vieleckig, insbesondere dreieckig, viereckig, fünfeckig, sechseckig, sternförmig, oval ist, einen Innenvielrund (102) definiert oder kreisförmig mit mindestens einer Abflachung.

13. Medizinisches Instrument nach einem der voranstehenden Ansprüche, **dadurch gekennzeichnet, dass**
a) die erste Branche (12) mindestens ein erstes Werkzeugelement (22) umfasst, dass die zweite Branche (14) mindestens ein zweites Werkzeugelement (24) umfasst und dass das mindestens eine erste Werkzeugelement (22) und das mindestens eine zweite Werkzeugelement (24) miteinander zusammenwirkend angeordnet sind zur Ausbildung eines medizinischen Werkzeugs (104),
wobei insbesondere das mindestens eine erste Werkzeugelement (22) in Form einer Schneide (106) oder eines Klemmbackens ausgebildet ist und/oder dass das mindestens eine zweite Werkzeugelement (24) in Form einer Schneide (108) oder eines Klemmbackens ausgebildet ist,
und/oder
b) das medizinische Instrument (10) in Form einer Fasszange, einer Schere, eines Nadelhalter oder einer Klemme ausgebildet ist.

14. Verfahren zum Herstellen eines medizinischen Instruments (10) mit einer ersten Branche (12) und einer zweiten Branche (14), die in einer Arbeitsstellung des Instruments (10) miteinander gekoppelt und um eine gemeinsame Schwenkachse (18) relativ zueinander verschwenkbar sind, wobei die erste Branche (12) und die zweite Branche (14) in einer Montagestellung voneinander getrennt sind und mit einer Schlussschraube (16) miteinander gekoppelt werden, wobei eine Längsachse (50) der Schlussschraube (16) die Schwenkachse (18) definiert, **dadurch gekennzeichnet, dass** zum Koppeln der ersten Branche (12) und der zweiten Branche (14) eine Schlussschraube (16) eingesetzt wird, an der ein von dieser in der Montagestellung abstehendes Einschraubelement (68) angeordnet oder ausgebildet ist, dass das Einschraubelement (68) mindestens eine Einschraubwerkzeugkontaktfläche (70) zum kraft- und/oder formschlüssigen in Eingriff Bringen mit einem Einschraubwerkzeug (72) aufweist, dass zum Koppeln der ersten Branche (12) und der zweiten Branche (14) mit der Schlussschraube (16) das Einschraubwerkzeug (72) mit der mindestens einen Einschraubwerkzeugkontaktfläche (70) kraft- und/oder formschlüssig in Eingriff gebracht wird und dass das Einschraubelement (68) nach dem Koppeln der ersten Branche (12) und der zweiten Branche (14) von der Schlussschraube (16) abgetrennt wird.

15. Verwendung eines Verfahrens nach Anspruch 14 zum Herstellen eines medizinischen Instruments (10) nach einem der Ansprüche 1 bis 13.

## Claims

1. Medical instrument (10) having a first branch (12) and a second branch (14), which in a working position of the instrument (10) are coupled to one another by a connecting screw (16) and are pivotable relative to one another about a common pivot axis (18), wherein a longitudinal axis (20) of the connecting screw (16) defines the pivot axis (18), **characterized in that** arranged or formed on the connecting screw (16) is a screw-in element (68) projecting therefrom in an assembly position, **in that** the screw-in element (68) has at least one screw-in tool contact face (70) for being brought into force-locking and/or positive-locking engagement with a screw-in tool (72), and **in that** the screw-in element (68) is separable from the connecting screw (16) after coupling the first branch (12) and the second branch (14) to one another.

2. Medical instrument in accordance with Claim 1, **characterized in that** the connecting screw (16) and the screw-in element (68) are of one piece, in particular monolithic, configuration.

3. Medical instrument in accordance with any one of the preceding Claims, **characterized in that** the screw-in element (68) is arranged or formed on the connecting screw (16) in such a way that the longitudinal axis (50) passes through the screw-in element (68).

4. Medical instrument in accordance with any one of the preceding Claims, **characterized in that** a predetermined breaking point (74) for separating the screw-in element (68) from the connecting screw (16) is formed between the screw-in element (68) and the connecting screw (16), wherein, in particular, the predetermined breaking point (74) defines an outer cross sectional area (76) transverse, in particular perpendicular, to the longitudinal axis (50), which is smaller than a minimum external cross sectional area (78) of the screw-in element (68) and smaller than a minimum external cross sectional area (80; 82) of the connecting screw (16).

5. Medical instrument in accordance with any one of the preceding Claims, **characterized in that** the connecting screw (16) comprises a screw head (38) and a bolt (42) projecting from the screw head (38), and **in that** the bolt (42) has at least one external thread portion (44), which extends at least over part of a length of the bolt (42) in parallel to the longitudinal axis (50), in particular over the entire length of the bolt (42).

6. Medical instrument in accordance with Claim 5, **characterized in that** the first branch (12) has a first perforation (52), **in that** the second branch (14) has a second perforation (54), **in that** an internal thread (56) that corresponds to the external thread portion (44) is formed on the second perforation (54), and **in that** in the working position of the medical instrument (10) the bolt (42) passes through the first perforation (52), the external thread portion (44) is screwed to the internal thread (56), and the screw head (38) abuts against a side face (88) of the first branch (12) that faces away from the second branch (14).

7. Medical instrument in accordance with Claim 6, **characterized in that**
a) the screw-in body (68) is dimensioned in such a way that it can be passed through the first and the second perforation (52, 54),
and/or
b) arranged on the first branch (12) is a screw head recess (58) that is arranged coaxially to the first perforation (52) and is widened in cross section in relation to the first perforation for at least partially, in particular completely, accommodating the screw head (38).

8. Medical instrument in accordance with any one of Claims 5 to 7, **characterized in that** the screw head (38) defines a screw head outer diameter (60) transverse to the longitudinal axis (50), **in that** the bolt (42) defines a bolt outer diameter (64) transverse to the longitudinal axis (50), and **in that** the screw head outer diameter (60) is greater than the bolt outer diameter (64).

9. Medical instrument in accordance with any one of Claims 5 to 8, **characterized in that** the screw head (38) defines a screw head end face (66) that points away from the bolt (42),
wherein, in particular,
a) the screw-in element (68) is arranged or formed projecting from the screw head end face (66)
and/or
b) the screw head end face (66) is of smooth or substantially smooth configuration, in particular in the assembly position or after separating the screw-in element (68) from the connecting screw (66)
and/or
c) the screw head end face (66) is of planar configuration or is formed convexly or concavely curved facing away from the screw head (38)
and/or
d) the screw head end face (66) extends transversely, in particular perpendicularly, to the longitudinal axis (50),
and/or
e) in the working position the screw head end face (66) is arranged flush with a side face of the first branch (12) that faces away from the second branch (14), in particular after separating off the screw-in element (68).

10. Medical instrument in accordance with any one of Claims 5 to 9, **characterized in that** the bolt (42) defines a bolt end face (48) pointing away from the screw head (38),
wherein, in particular,
a) the screw-in element (68) is arranged or formed projecting from the bolt end face (48)
and/or
b) the bolt end face (48) is of smooth or substantially smooth configuration, in particular in the assembly position or after separating the screw-in element (68) from the connecting screw (16),
and/or
c) the bolt end face (48) is of planar configuration or is formed convexly or concavely curved facing away from the bolt (42)
and/or
d) the bolt end face (48) extends transversely, in particular perpendicularly, to the longitudinal axis (50)
and/or
e) in the working position the bolt end face (48) is arranged flush with a side face (86) of the second branch (14) that faces away from the first branch (12), in particular after separating off the screw-in element (68).

11. Medical instrument in accordance with any one of the preceding Claims, **characterized in that**
a) the at least one screw-in tool contact face (70) is formed facing away from or toward the pivot axis (18)
and/or
b) the screw-in element (68) is of symmetric configuration in relation to the longitudinal axis (50) or in relation to a plane containing the longitudinal axis (50)
and/or
c) the screw-in element (68) in the region of the at least one screw-in tool contact face (70) defines an external cross sectional area transverse, in particular perpendicular, to the longitudinal axis (50), and **in that** the cross sectional area is circular or has a shape deviating from a circular shape,
wherein, in particular, the shape of the external cross sectional area deviating from a circular shape is polygonal, in particular triangular, quadrangular, pentagonal, hexagonal, star-shaped, oval, defines an external multilobular profile (92), or is circular with at least one flattened portion (90).

12. Medical instrument in accordance with any one of the preceding Claims, **characterized in that** the screw-in element (68) defines a receptacle (94) for a screw-in tool (72) and **in that** the receptacle (94) defines the at least one screw-in tool contact face (70),
wherein, in particular, the receptacle (94)
a) is configured to be brought into force-locking and/or positive-locking engagement with a screw-in tool (72)
and/or
b) is configured in the form of a slit (96) or a recess (98), in particular in the form of a blind hole (100)
and/or
c) defines an internal cross sectional area in the region of the at least one screw-in tool contact face (70) transverse, in particular perpendicular, to the longitudinal axis (50), and **in that** the internal cross sectional area is circular or has a shape deviating from a circular shape,
wherein, in particular, the shape of the internal cross sectional area deviating from a circular shape is polygonal, in particular triangular, quadrangular, pentagonal, hexagonal, star-shaped, oval, defines an internal multilobular profile (102), or is circular with at least one flattened portion.

13. Medical instrument in accordance with any one of the preceding Claims, **characterized in that**
a) the first branch (12) comprises at least one first tool element (22), **in that** the second branch (14) comprises at least one second tool element (24), and **in that** the at least one first tool element (22) and the at least one second tool element (24) are arranged cooperating with one another to form a medical tool (104), wherein, in particular, the at least one first tool element (22) is configured in the form of a blade (106) or a clamping jaw and/or **in that** the at least one second tool element (24) is configured in the form of a blade (108) or a clamping jaw,
and/or
b) the medical instrument (10) is configured in the form of gripping forceps, scissors, a needle holder, or a clamp.

14. Method for producing a medical instrument (10) having a first branch (12) and a second branch (14), which in a working position of the instrument (10) are coupled to one another and are pivotable relative to one another about a common pivot axis (18), wherein the first branch (12) and the second branch (14) in an assembly position are separated from one another and are coupled to one another with a connecting screw (16), wherein a longitudinal axis (50) of the connecting screw (16) defines the pivot axis (18), **characterized in that** for coupling the first branch (12) and the second branch (14), a connecting screw (16) is used, on which a screw-in element (68) projecting therefrom in the assembly position is arranged or formed, **in that** the screw-in element (68) has at least one screw-in tool contact face (70) for being brought into force-locking and/or positive-locking engagement with a screw-in tool (72), **in that** for coupling the first branch (12) and the second branch (14) with the connecting screw (16), the screw-in tool (72) is brought into force-locking and/or positive-locking engagement with the at least one screw-in tool contact face (70), and **in that** the screw-in element (68) is separated from the connecting screw (16) after the coupling of the first branch (12) and the second branch (14).

15. Use of a method in accordance with Claim 14 for producing a medical instrument (10) in accordance with any one of Claims 1 to 13.

## Revendications

1. Instrument médical (10) avec une première branche (12) et une deuxième branche (14), qui sont couplées l'une à l'autre dans une position de travail de l'instrument (10) avec une vis de fermeture (16) et peuvent être amenées à pivoter l'une par rapport à l'autre autour d'un axe de pivotement commun (18), où un axe longitudinal (20) de la vis de fermeture (16) définit l'axe de pivotement (18), **caractérisé en ce qu'**un élément de vissage (68) dépassant de la vis de fermeture (16) dans une position de montage est disposé ou conçu sur celle-ci, **en ce que** l'élément de vissage (68) présente au moins une face de contact avec un outil de vissage (70) pour l'engagement par assemblage de force et/ou de forme avec un outil de vissage (72) et **en ce que** l'élément de vissage (68) peut être séparé de la vis de fermeture (16) après le couplage de la première branche (12) et de la deuxième branche (14) l'une à l'autre.

2. Instrument médical selon la revendication 1, **caractérisé en ce que** la vis de fermeture (16) et l'élément de vissage (68) sont conçus d'une seule pièce, en particulier de manière monolithique.

3. Instrument médical selon l'une des revendications précédentes, **caractérisé en ce que** l'élément de vissage (68) est disposé ou conçu sur la vis de fermeture (16) de telle sorte que l'axe longitudinal (50) traverse l'élément de vissage (68).

4. Instrument médical selon l'une des revendications précédentes, **caractérisé en ce qu'**un point destiné à la rupture (74) pour séparer l'élément de vissage (68) de la vis de fermeture (16) est conçu entre l'élément de vissage (68) et la vis de fermeture (16),
où en particulier le point destiné à la rupture (74) définit une aire de section transversale externe (76) transversalement, en particulier perpendiculairement, à l'axe longitudinal (50), qui est plus petite qu'une aire de section transversale externe minimale (78) de l'élément de vissage (68) et plus petite qu'une aire de section transversale externe minimale (80; 82) de la vis de fermeture (16).

5. Instrument médical selon l'une des revendications précédentes, **caractérisé en ce que** la vis de fermeture (16) comprend une tête de vis (38) et un boulon (42) dépassant de la tête de vis (38) et **en ce que** le boulon (42) présente au moins une section de filetage externe (44) qui s'étend au moins sur une partie d'une longueur du boulon (42) parallèlement à l'axe longitudinal (50), en particulier sur toute la longueur du boulon (42).

6. Instrument médical selon la revendication 5, **caractérisé en ce que** la première branche (12) présente une première ouverture (52), **en ce que** la deuxième branche (14) présente une deuxième ouverture (54), **en ce qu'**au niveau de la deuxième ouverture (54) un filetage interne (56) est formé, qui correspond à la section de filetage externe (44), et **en ce que** dans la position de travail de l'instrument médical (10), le boulon (42) traverse la première ouverture (52), la section de filetage externe (44) est vissée avec le filetage interne (56) et la tête de vis (38) repose contre une face latérale (88) de la première branche (12) tournée à l'opposé de la deuxième branche (14).

7. Instrument médical selon la revendication 6, **caractérisé en ce que**
a) le corps de vissage (68) est dimensionné de telle sorte qu'il peut être amené à passer à travers la première et la deuxième ouverture (52, 54),
et/ou
b) sur la première branche (12) un évidement de tête de vis (58) disposé de manière coaxiale à la première ouverture (52) et élargi en section transversale par rapport à la première ouverture (52) est conçu pour recevoir au moins en partie, en particulier totalement, la tête de vis (38).

8. Instrument médical selon l'une des revendications 5 à 7, **caractérisé en ce que** la tête de vis (38) définit un diamètre externe de tête de vis (60) transversalement à l'axe longitudinal (50), **en ce que** le boulon (42) définit un diamètre externe de boulon (64) transversalement à l'axe longitudinal (50) et **en ce que** le diamètre externe de tête de vis (60) est supérieur au diamètre externe de boulon (64).

9. Instrument médical selon l'une des revendications 5 à 8, **caractérisé en ce que** la tête de vis (38) définit une face d'extrémité de tête de vis (66) tournée à l'opposé du boulon (42),
où en particulier
a) l'élément de vissage (68) est disposé ou conçu dépassant de la face d'extrémité de tête de vis (66)
et/ou
b) la face d'extrémité de tête de vis (66) est conçue lisse ou sensiblement lisse, en particulier dans la position de montage ou après la séparation de l'élément de vissage (68) de la vis de fermeture (66)
et/ou
c) la face d'extrémité de tête de vis (66) est conçue plane ou incurvée de manière convexe ou concave à l'opposé de la tête de vis (38)
et/ou
d) la face d'extrémité de tête de vis (66) s'étend transversalement, en particulier perpendiculairement, à l'axe longitudinal (50),
et/ou
e) dans la position de travail, la face d'extrémité de tête de vis (66) est disposée à fleur de surface d'une face latérale de la première branche (12) tournée à l'opposé de la deuxième branche (14), en particulier après la séparation de l'élément de vissage (68).

10. Instrument médical selon l'une des revendications 5 à 9, **caractérisé en ce que** le boulon (42) définit une face d'extrémité de boulon (48) tournée à l'opposé de la tête de vis (38),
où en particulier
a) l'élément de vissage (68) est disposé ou conçu dépassant de la face d'extrémité de boulon (48)
et/ou
b) la face d'extrémité de boulon (48) est conçue lisse ou sensiblement lisse, en particulier dans la position de montage ou après la séparation de l'élément de vissage (68) de la vis de fermeture (16),
et/ou
c) la face d'extrémité de boulon (48) est conçue plane ou incurvée de manière convexe ou concave à l'opposé du boulon (42),
et/ou
d) la face d'extrémité de boulon (48) s'étend transversalement, en particulier perpendiculairement, à l'axe longitudinal (50),
et/ou
e) dans la position de travail, la face d'extrémité de boulon (48) est disposée à fleur de surface d'une face latérale (86) de la deuxième branche (14) tournée à l'opposé de la première branche (12), en particulier après la séparation de l'élément de vissage (68).

11. Instrument médical selon l'une des revendications précédentes,
**caractérisé en ce que**
a) la au moins une face de contact avec un outil de vissage (70) est conçue tournée à l'opposé de l'axe de pivotement (18) ou vers celui-ci
et/ou
b) l'élément de vissage (68) est conçu symétriquement par rapport à l'axe longitudinal (50) ou par rapport à un plan contenant l'axe longitudinal (50),
et/ou
c) l'élément de vissage (68) définit, dans la zone de la au moins une face de contact avec un outil de vissage (70), une aire de section transversale externe transversalement, en particulier perpendiculairement, à l'axe longitudinal (50) et **en ce que** l'aire de section transversale est circulaire ou présente une forme s'écartant d'une forme circulaire,
où en particulier la forme s'écartant d'une forme circulaire de la aire de section transversale externe est polygonale, en particulier triangulaire, quadrangulaire, pentagonale, hexagonale, en forme d'étoile, ovale, définit un polyarrondi externe (92) ou circulaire avec au moins un aplatissement (90).

12. Instrument médical selon l'une des revendications précédentes, **caractérisé en ce que** l'élément de vissage (68) définit un logement (94) pour un outil de vissage (72) et **en ce que** le logement (94) définit la au moins une face de contact avec un outil de vissage (70),
où en particulier le logement (94)
a) est conçu pour un engagement par assemblage de force et/ou de forme avec un outil de vissage (72)
et/ou
b) est conçu sous la forme d'une fente (96) ou d'un évidement (98), en particulier sous la forme d'un trou borgne (100),
et/ou
c) définit une aire de section transversale interne dans la zone de la au moins une face de contact avec un outil de vissage (70) transversalement, en particulier perpendiculairement, à l'axe longitudinal (50) et **en ce que** l'aire de section transversale interne est circulaire ou présente une forme s'écartant d'une forme circulaire,
où en particulier la forme de la aire de section transversale interne s'écartant d'une forme circulaire est polygonale, en particulier triangulaire, quadrangulaire, pentagonale, hexagonale, en forme d'étoile, ovale, définit un polyarrondi interne (102) ou circulaire avec au moins un aplatissement.

13. Instrument médical selon l'une des revendications précédentes, **caractérisé en ce que**
a) la première branche (12) comprend au moins un premier élément d'outil (22), **en ce que** la deuxième branche (14) comprend au moins un deuxième élément d'outil (24) et **en ce que** le au moins un premier élément d'outil (22) et le au moins un deuxième élément d'outil (24) sont disposés coopérant l'un avec l'autre pour former un outil médical (104),
où en particulier le au moins un premier élément d'outil (22) est conçu sous la forme d'une lame (106) ou d'une mâchoire de serrage et/ou **en ce que** le au moins un deuxième élément d'outil (24) est conçu sous la forme d'une lame (108) ou d'une mâchoire de serrage,
et/ou
b) l'instrument médical (10) est conçu sous forme d'un forceps, de ciseaux, d'un porte-aiguille ou d'une pince.

14. Procédé pour la production d'un instrument médical (10) avec une première branche (12) et une deuxième branche (14), qui sont couplées l'une à l'autre dans une position de travail de l'instrument (10) et peuvent être amenées à pivoter l'une par rapport à l'autre autour d'un axe de pivotement commun (18), où la première branche (12) et la deuxième branche (14) sont séparées l'une de l'autre dans une position de montage et sont couplées l'une à l'autre avec une vis de fermeture (16), où un axe longitudinal (50) de la vis de fermeture (16) définit l'axe de pivotement (18), **caractérisé en ce qu'**une vis de fermeture (16) est utilisée pour le couplage de la première branche (12) et de la deuxième branche (14), sur laquelle est disposé ou conçu un élément de vissage (68) dépassant de celle-ci dans la position de montage, **en ce que** l'élément de vissage (68) présente au moins une face de contact avec un outil de vissage (70) pour l'engagement par assemblage de force et/ou de forme avec un outil de vissage (72), **en ce que**, pour le couplage de la première branche (12) et de la deuxième branche (14) avec la vis de fermeture (16), l'outil de vissage (72) est amené en engagement avec la au moins une face de contact avec un outil de vissage (70) par assemblage de force et/ou de forme et **en ce que** l'élément de vissage (68) est séparé de la vis de fermeture (16) après le couplage de la première branche (12) et de la deuxième branche (14).

15. Utilisation d'un procédé selon la revendication 14 pour la production d'un instrument médical (10) selon l'une des revendications 1 à 13.
